# EUROPEAN PATENT SPECIFICATION

(11) **EP 1 837 000 B1**
(45) Date of publication and mention of the grant of the patent: **26.11.2008**
(21) Application number: 06425160.6
(22) Date of filing: 09.03.2006
(51) Int. Cl.: A61F 5/00

(54) **Adjustment device for knee brace or orthopedic walker uprights**
Verstellvorrichtung für eine Knieorthese oder für eine orthopädische Gehhilfe
Dispositif de réglage pour une orthèse de genou ou pour un déambulateur orthopédique

(43) Date of publication of application: 26.09.2007
(73) Proprietor: F.G.P. Srl, 37062 Dossobuono (VR) (IT)
(72) Inventor: Turrini, Alberto, 37062 Castel d'Azzano (VR) (IT); Ferrigolo, Moreno, 37062 Dossobuono (VR) (IT)
(74) Representative: Petraz, Gilberto Luigi

(56) References cited:
- EP-A- 0 553 523
- EP-A- 1 366 865
- DE-A1- 10 027 795
- FR-A- 2 776 507
- US-A- 5 242 378
- US-B1- 6 610 090
- US-B1- 6 821 233

## Description

### TECHNICAL FIELD

This invention concerns an adjustment device which can be used in particular for postoperative orthopedic knee brace uprights.

More specifically, this invention refers to an adjustment device for postoperative orthopedic knee brace uprights with the particular feature of consisting of means that allow the extension or length adjustment of the uprights of the orthopedic braces.

This device allows the orthopedic brace to be used in a modular way, that is by subjects with different body size or if it is necessary to extend the brace during rehabilitation.

This invention can be applied in the orthopedic-medical industry with particular reference to manufacturers of prostheses and braces.

### BACKGROUND ART

The use is known in the orthopedic-medical sector of orthopedic knee braces equipped with various devices for adjustment of the angular excursion to ensure a correct end-of-stroke both when the tibia is extended and when the tibia is flexed with respect to the femur.

According to the known technique, traditional knee braces usually consist of a rigid frame which encloses the leg in order to guarantee sufficient support to prevent the onset of strain on the knee joint for the injured and/or convalescent person when walking.

The knee brace frame generally comprises means for restraining it to the femur and the tibia close to the knee and a connecting structure of these means with an articulated joint at the level of the knee.

The knee brace frame comprises uprights, positioned laterally with respect to the femur and the tibia, connected by respective joints generally equipped with 4 pivots designed to ensure appropriate mobility by means of the presence of multiple rotation centres.

To ensure sufficient freedom of movement of the limb, the frame develops almost exclusively at the sides of the knee in order to allow correct reciprocal oscillation between the femur and the tibia, in extension and flexion directions.

The means of restraint usually consist of half-rings enclosing both the femur and the tibia of the injured person.

The four-pivot articulated joints are used to ensure the hinged connection of parts of the frame fixed to the femur and the tibia and extend during flexion of the limb and tend to shorten when the limb is extended.

This considerably reduces the risk of movements of the knee brace and of its slipping downwards.

A drawback of known orthopedic braces is that they are equipped with uprights that do not foresee means of adjustment as regards length and are therefore unlikely to allow the device to be adapted to various people who may also have different body sizes, or to different types of trauma, thus often making it necessary to use different forms of orthosis.

The most typical case may in fact occur whereby the brace is bought for one person and cannot be used by another person, for example, a member of the same family, only because the second person is a different body size.

Similarly, and more in general, the orthopedic braces may be owned by a rehabilitation centre and thus be used by various patients, all different sizes, making it necessary to have braces of various shapes and sizes.

Another drawback is that a traditional orthopedic knee brace, not being equipped with upright adjustment means, is unlikely to be adaptable to various types of injury or rehabilitation, for example if it is necessary to employ various ways of positioning the brace on the limb to be rehabilitated.

Document FR-A-2 776 507 discloses an adjustment device for extending and reducing the length of a knee brace according to the preamble of claim 1.

### DESCRIPTION OF THE INVENTION

This invention proposes to provide an adjustment device that can be used in particular for postoperative orthopedic knee brace uprights and which is able to eliminate or at least reduce the drawbacks described above and other immediately consequent drawbacks.

This invention also proposes to provide an adjustment device that can be used in particular for postoperative orthopedic knee brace uprights needing extension adjustments of the brace, that is to say when the same brace must be used for persons of different body size.

This is achieved by means of an adjustment device that can be used in particular for postoperative orthopedic knee brace uprights with the features described in the main claim.

The dependent claims describe advantageous embodiments of the invention.

The adjustment device according to the invention, particularly appropriate to allow the extension and reduction in length of postoperative orthopedic knee brace uprights, foresees that each upright consists of a mobile external part and a fixed inner part placed close to the knee.

The mobile outer part of each upright is equipped with at least one substantially quadrilateral opening with toothed edges, in which a cursor with flexible segments slides, forming an element that allows the blocking and/or release of the movement of the mobile part of the upright with respect to the fixed part.

The device also comprises a safety slide, controlled manually, equipped with a prong that, in the blocked position, is wedged between the flexible blades of the cursor, allowing or preventing the movement inside the opening of the outer upright according to whether the prong is in the blocked or free position.

According to the invention, when it is necessary to adjust the length of one or more uprights, it is sufficient to move the slide towards the centre of the knee brace in order to release the cursor, thus allowing the movement and adjustment of the upright concerned.

As these operations require only the pressure and movement of the slide for a short distance, the adjustment of the knee brace uprights according to the invention is extremely simple and does not require the intervention of specialised personnel.

The device described above can be made from lightweight metal alloy or from high resistance composite plastic.

### DESCRIPTION OF THE DRAWINGS

Other features and advantages of the invention will become evident on reading the following description of one embodiment of the invention, given as a non-binding example, with the help of the accompanying drawings in which:
- figure 1 represents a schematic prospective view of an adjustment device for knee brace uprights according to the invention;
- figure 2 shows a prospective exploded view of the adjustment device;
- figure 3 is a detailed schematic view of a cursor element according to a possible construction variation;

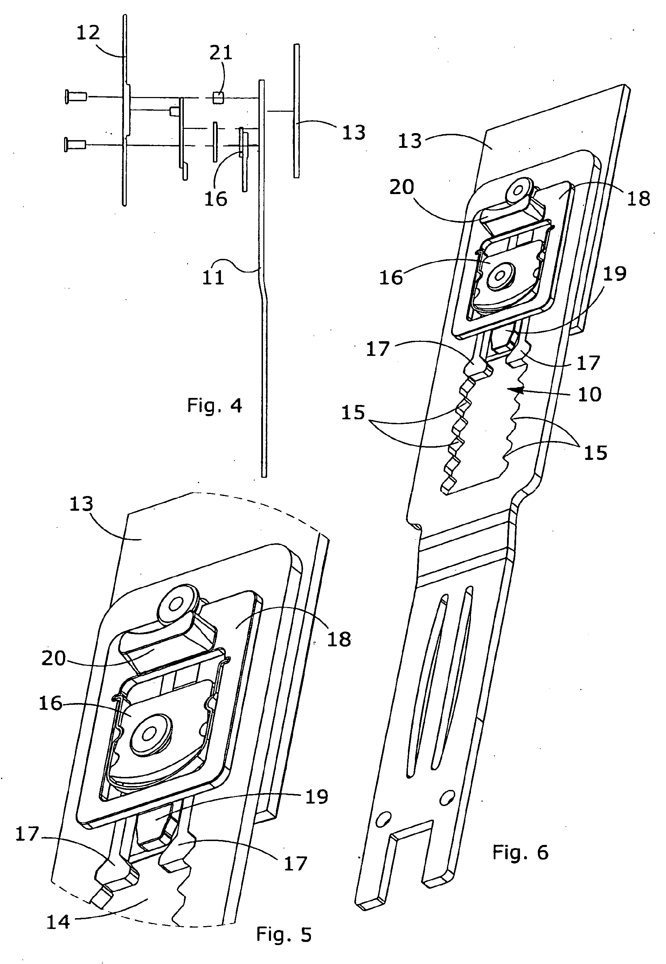

- figure 4 is a schematic exploded side view of the components of the device according to the invention; and
- figure 5 shows a schematic view of the internal mechanical part of the adjustment device;
- figure 6 shows a detail of the mechanical device for blocking and releasing the upright.

### DESCRIPTION OF ONE EMBODIMENT

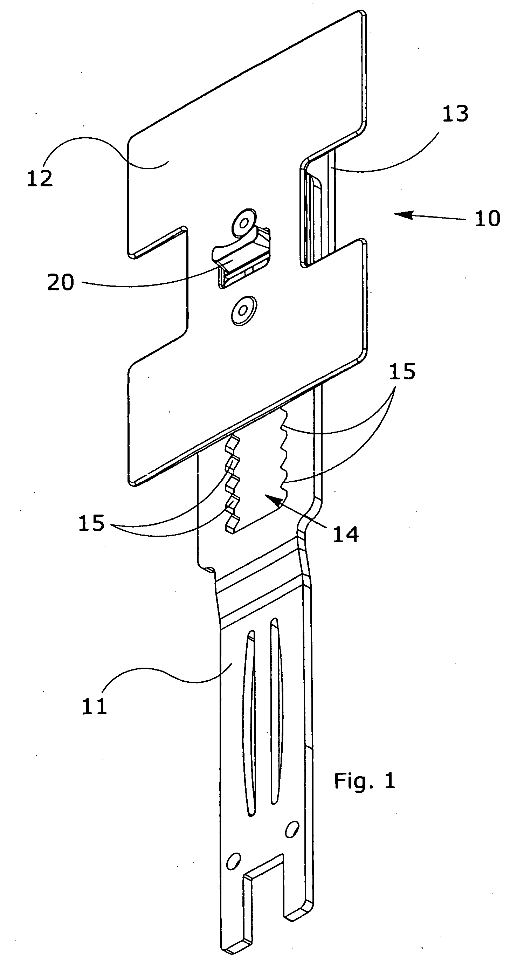

With reference first of all to figure 1, the reference number 10 indicates in general an adjustment device according to the invention, the use of which allows the extension and reduction in length of postoperative orthopedic knee brace uprights.

The adjustment device according to the invention foresees, therefore, that each upright consists of a mobile outer part 11 and a fixed inner part placed close to the knee.

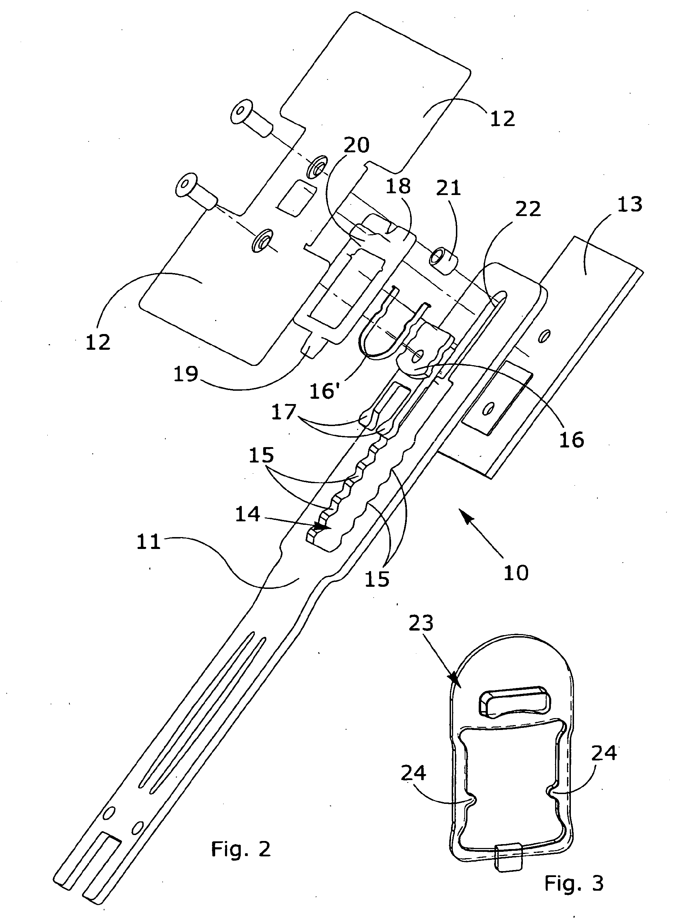

Figures 1 and 2 show only the fixed inner part coupling area, consisting of a plate 12 placed opposite a blocking plate 13 positioned on the inner side of the brace.

The mobile outer part 11 of each upright is equipped with at least one substantially quadrilateral opening 14 with toothed edges 15, in which a cursor 16 slides, this cursor being equipped with opposite flexible blades 17 with enlarged appendages at their ends designed to be housed in the cavities of the toothed edges 15.

The adjustment device according to the invention also comprises a safety slide 18, substantially quadrilateral in shape and equipped with a prong 19 shaped in such a way that it wedges between the two flexible blades 17 of the cursor 16.

The cursor-slide unit is equipped with an interposed elastic tab 16', made from music wire, which allows the structure to remain constantly in working order.

The slide 18 is controlled by hand and is thus equipped with a knob 20 that can be operated with the fingers.

In the blocked position, the prong 19 is wedged between the two flexible blades 17 of the cursor 16, thus allowing or preventing the movement inside the opening 14 of the outer upright 11 according to whether the prong is in the blocked or free position.

The cursor 16 thus forms an element that allows the blocking and/or release of the movement of the mobile part of the upright 11 with respect to the fixed part.

According to the invention, when it is necessary to adjust the length of one or both the uprights, it is sufficient to move the slide 18 towards the centre of the knee brace, that is to say in the opposite direction to the upright to be released, in order to release the respective cursor 16, thus allowing the movement and adjustment of the upright concerned.

In fact, once the enlarged appendages of the two flexible blades 17 are released, the upright 11 can slide longitudinally, helped by a fixed plug 21 which slides inside a slot 22 cut in the end of the upright 11.

As these operations require only the pressure and movement of the slide for a short distance, the adjustment of the uprights according to the invention is extremely simple and does not require the help of specialised personnel.

Figure 3 shows a variation which foresees the possibility of constructing the slide 18 and the music wire elastic tab 16', in a single body 23. In this case the slide 23 has an internal opening with a pair of opposite projections 24 which carry out the same function as the elastic tab, engaging with the shaped cursor 16.

The articulated joint 10 can be made from lightweight metal alloy or from high resistance composite plastic.

The invention is described above with reference to a preferred embodiment. It is nevertheless clear that the invention is susceptible to numerous variations that lie within the framework of technical equivalents.

## Claims

1. An adjustment device (10) for extending and reducing the length of postoperative orthopedic knee brace uprights, comprising a mobile outer part (11) with respect to a fixed inner part placed close to the knee where a fixing plate (12) is positioned opposite a blocking plate (13) on the inner side of the brace, **characterised in that** the mobile outer part (11) of each upright is provided with a substantially quadrilateral opening (14) with toothed edges (15), in which a cursor (16) slides and is provided with opposite flexible blades (17) the ends of which have enlarged appendages which engage with the cavities in the toothed edges (15) and **in that** it comprises a safety slide (18), equipped with a prong (19) shaped in such a way that it wedges between the two flexible blades (17) of the cursor (16).

2. An adjustment device (10) according to the foregoing claim, **characterised in that** the slide (18) is controlled by hand and is therefore equipped with a knob (20) which can be operated with the fingers.

3. An adjustment device (10) according to either of the foregoing claims, **characterised in that** in the blocked position the prong (19) is wedged between the two flexible blades (17) of the cursor (16), allowing or preventing its movement inside the opening (14) in the outer upright (11) according to whether the prong is in the blocked or free position.

4. An adjustment device (10) according to any of the foregoing claims, **characterised in that** the cursor (16) forms an element that allows the blocking and/or release of the movement of the mobile part (11) of the upright with respect to the fixed part.

5. An adjustment device (10) according to any of the foregoing claims, **characterised in that** the cursor-slide unit is equipped with an interposed elastic tab (16'), made from music wire, which allows the structure to remain in constantly working order.

6. An adjustment device (10) according to any of the foregoing claims, **characterised in that** the slide (18) and the music wire elastic tab (16') are made in a single body (23) and in this case the element (23) presents an opening with a pair of opposite projections (24) which have the same function as the elastic tab, engaging with the shaped cursor (16).

## Patentansprüche

1. Einstellvorrichtung (10) zum Vergrößern und Verringern der Länge von postoperativen orthopädischen Knieorthesenständern, die einen in Bezug auf einen unbeweglichen inneren Teil beweglichen äußeren Teil (11) umfasst, der nahe dem Knie angeordnet ist, wo eine Fixierungsplatte (12) gegenüber einer Blockierungsplatte (13) auf der inneren Seite der Orthese angeordnet ist, **dadurch gekennzeichnet, dass** der bewegliche äußere Teil (11) jedes Ständers mit einer im Wesentlichen vierseitigen Öffnung (14) mit gezahnten Kanten (15) versehen ist, in der ein Positionsanzeiger (16) gleitet und mit einander gegenüberliegenden flexiblen Zungen (17) versehen ist, deren Enden vergrößerte Anhänge haben, die mit den Hohlräumen in den gezahnten Kanten (15) ineinandergreifen, und **dadurch**, dass sie ein Sicherheitsgleitstück (18) umfasst, das mit einer Zacke (19) ausgestattet ist, die auf eine solche Weise geformt ist, dass sie sich zwischen den zwei flexiblen Zungen (17) des Positionsanzeigers (16) verkeilt.

2. Einstellvorrichtung (10) nach dem vorhergehenden Anspruch, **dadurch gekennzeichnet, dass** das Gleitstück (18) mit der Hand gesteuert wird und deshalb mit einem Knopf (20) ausgestattet ist, der mit dem Fingern bedient werden kann.

3. Einstellvorrichtung (10) nach einem der vorhergehenden Ansprüche, **dadurch gekennzeichnet, dass** in der blockierten Position die Zacke (19) zwischen den zwei flexiblen Zungen (17) des Positionsanzeigers (16) verkeilt wird, was es ermöglicht, dessen Bewegung innerhalb der Öffnung (14) in dem äußeren Ständer (11) zu verhindern, je nachdem, ob sich die Zacke in der blockierten oder der freien Position befindet.

4. Einstellvorrichtung (10) nach einem der vorhergehenden Ansprüche, **dadurch gekennzeichnet, dass** der Positionsanzeiger (16) ein Element bildet, welches das Blockieren und/oder das Freigeben der Bewegung des beweglichen Teils (11) des Ständers in Bezug auf den unbeweglichen Teil ermöglicht.

5. Einstellvorrichtung (10) nach einem der vorhergehenden Ansprüche, **dadurch gekennzeichnet, dass** die Einheit von Positionsanzeiger und Gleitstück mit einem dazwischengefügten elastischen Streifen (16'), hergestellt aus Saitendraht, ausgestattet ist, der es ermöglicht, dass die Struktur in einem gleichbleibenden Betriebszustand bleibt.

6. Einstellvorrichtung (10) nach einem der vorhergehenden Ansprüche, **dadurch gekennzeichnet, dass** das Gleitstück (18) und der elastische Seitendrahtstreifen (16') in einem einzigen Körper (23) hergestellt sind und in diesem Fall das Element (23) eine Öffnung mit einem Paar von einander gegenüberliegenden Vorsprüngen (24) darbietet, welche die gleiche Funktion haben wie der elastische Streifen, indem sie mit dem geformten Positionsanzeiger (16) ineinandergreifen.

## Revendications

1. Dispositif de réglage (10) permettant d'augmenter et de réduire la longueur des montants d'une genouillère orthopédique post-opératoire comprenant une partie extérieure (11) mobile par rapport à une partie intérieure fixe à proximité du genou où une plaque de fixation (12) est positionnée à l'opposé d'une plaque de blocage (13) se trouvant du côté intérieur de la genouillère, **caractérisé en ce que** la partie extérieure mobile (11) de chaque montant est dotée d'une ouverture (14), sensiblement quadrilatérale munie de bords dentés (15), dans lesquels un curseur (16) coulisse et est doté de lames flexibles opposées (17) dont les extrémités présentent des renflements qui s'engagent dans les cavités ménagées dans les bords dentés (15), et **en ce qu'**il comprend un coulisseau de sécurité (18), doté d'une saillie (19) conformée de manière à se coincer entre les deux lames flexibles (17) du curseur (16).

2. Dispositif de réglage (10) selon la revendication précédente, **caractérisée en ce que** le coulisseau (18) est commandé manuellement et est donc doté d'un bouton (20) qui peut être actionné avec les doigts.

3. Dispositif de réglage (10) selon l'une ou l'autre des revendications précédentes, **caractérisé en ce que**, dans la position bloquée, la saillie (19) est coincée entre les deux lames flexibles (17) du curseur (16), autorisant ou interdisant le mouvement de celui-ci à l'intérieur de l'ouverture (14) ménagée dans le montant extérieur (11) selon que la saillie se trouve dans la position bloquée ou dans la position libre.

4. Dispositif de réglage (10) selon l'une quelconque des revendications précédentes, **caractérisé en ce que** le curseur (16) forme un élément qui autorise le blocage et/ou la libération du mouvement de la partie mobile (11) du montant par rapport à la partie fixe.

5. Dispositif de réglage (10) selon l'une quelconque des revendications précédentes, **caractérisé en ce que** l'unité curseur/coulisseau est équipée d'une languette élastique interposée (16') qui est fabriquée à partir d'une corde à piano et qui permet à la structure de rester en permanence en position de travail.

6. Dispositif de réglage (10) selon l'une quelconque des revendications précédentes, **caractérisé en ce que** le coulisseau (18) et la languette élastique (16') en corde à piano sont fabriqués en un seul corps (23) et l'élément (23) présente alors une ouverture dotée d'une paire de saillies opposées (24) dont la fonction est identique à celle de la languette élastique, s'engagant avec le curseur (16) de forme adaptée.
